# EUROPEAN PATENT SPECIFICATION

(11) **EP 3 795 126 B1**
(45) Date of publication and mention of the grant of the patent: **10.05.2023**
(21) Application number: 19827455.7
(22) Date of filing: 18.06.2019
(51) Int. Cl.: A61F 13/494, A61F 13/53

(54) **ABSORBENT ARTICLE**
ABSORBIERENDER ARTIKEL
ARTICLE ABSORBANT

(30) Priority: 29.06.2018 JP 2018124775
(43) Date of publication of application: 24.03.2021
(73) Proprietor: Unicharm Corporation, Ehime 799-0111 (JP)
(72) Inventor: NAGAI, Takahito, Kanonji-shi, Kagawa 769-1602 (JP); TANAKA, Suguru, Kanonji-shi, Kagawa 769-1602 (JP)
(74) Representative: Dolleymores
(86) International application number: PCT/JP2019/024130
(87) International publication number: WO 2020/004150

(56) References cited:
- JP-A- 2008 302 138
- JP-A- 2010 094 334
- JP-A- 2014 180 345
- JP-A- 2019 118 569

## Description

### [Technical Field]

The present invention relates to an absorbent article.

### [Background Art]

As underpants-shaped absorbent articles, underpants-shaped disposable diapers are known. Patent Document 1 discloses an underpants-shaped disposable diaper including an outer sheet member, an absorbent body joined to a skin surface side of the outer sheet member, and side sheets respectively extending from two widthwise end portions of the absorbent body. In the diaper of Patent Literature 1, raising elastic members (38 and 39) for raising the side sheets are attached in a stretched state to the side sheets throughout the entire length of the side sheets in the longitudinal direction of the absorbent body. In addition, the side sheets rise toward the skin side of a wearer due to the contraction of the raising elastic members and function as so-called leakproof walls.

### [Patent Literature]

Patent Literature 1: Japanese Patent Application Publication No. 2010-94334

### [Summary of the Invention]

### [Technical Problem]

In the diaper of Patent Literature 1, fixing portions (joining portions 41c) that fix the side sheets to a top sheet of the absorbent body are provided at longitudinal middle portions of the side sheets (leakproof walls). In the portions where the fixing portions are provided, the absorbent body is pulled up toward the skin side by the contractive force that is generated by the raising elastic members, making it more likely to cause the absorbent body to deform. In particular, in a case where the pair of fixing portions (joining portions 41c) provided on two lateral sides are provided at the same position in the longitudinal direction of the absorbent body, a folding guide line extending along the lateral direction is formed between the fixing portions, making it more likely to cause the absorbent body to deform along the folding guide line into a shape protruding toward the skin side. In this case, there is a risk that a gap is formed between the absorbent body and the skin of the wearer, deteriorating the fit or absorption property of the diaper.

The present invention was achieved in light of conventional problems such as that described above, and an aspect of the present invention is to suppress the absorbent body deforming to protrude toward the skin side.

### [Solution to Problem]

A main aspect of the present invention for achieving the above-described aspect is an absorbent article including: an absorbent body including an absorbent core and a skin-side sheet disposed on a skin side with respect to the absorbent core; and a pair of leakproof wall portions including a stretching/contracting member that stretches and contracts, in a state in which the absorbent article is unfolded, the absorbent body having a front-back direction and a lateral direction that intersect each other, the pair of leakproof wall portions being provided on two lateral sides of the absorbent body, a front fixing portion being provided in a front end portion of the absorbent body and fixing the leakproof wall portions so that the leakproof wall portions are incapable of rising, a back fixing portion being provided in a back end portion of the absorbent body and fixing the leakproof wall portions so that the leakproof wall portions are incapable of rising, an intermediate fixing portion being provided between the front fixing portion and the back fixing portion in the front-back direction, the intermediate fixing portion fixing the leakproof wall portions to the skin-side sheet, the intermediate fixing portion being provided at a position apart from the front fixing portion and the back fixing portion in the front-back direction, the intermediate fixing portion having a first intermediate fixing portion and a second intermediate fixing portion, the first intermediate fixing portion being disposed on a lateral one side, the second intermediate fixing portion being disposed on a lateral other side, a position of a front end of the first intermediate fixing portion and a position of a front end of the second intermediate fixing portion being different in the front-back direction. Other features of the present invention will be further clarified by the description of the present specification and the accompanying drawings.

### [Advantageous Effect of the Invention]

According to the present invention, it is possible to suppress the absorbent body deforming to protrude toward the skin side.

### [Brief Description of Drawings]

FIG. 1 is a schematic front view of a diaper 1.
FIG. 2 is a schematic plan view of the diaper 1 in a stretched state.
FIG. 3 is a schematic cross-sectional view taken along a line I-I in FIG. 2.
FIG. 4 is an enlarged plan view illustrating the disposition of intermediate fixing portions 53.
FIG. 5 is an enlarged plan view illustrating the disposition of intermediate fixing portions 53 in a diaper 2 of a comparative example.
FIG. 6 is a schematic cross-sectional view of the diaper 2 in an underpants-shaped state, cut in the front-back direction.

### [Description of Embodiments]

Description of the present specification and the accompanying drawings clarifies at least the following matters.

An absorbent article including: an absorbent body including an absorbent core and a skin-side sheet disposed on a skin side with respect to the absorbent core; and a pair of leakproof wall portions including a stretching/contracting member that stretches and contracts, in a state in which the absorbent article is unfolded, the absorbent body having a front-back direction and a lateral direction that intersect each other, the pair of leakproof wall portions being provided on two lateral sides of the absorbent body, a front fixing portion being provided in a front end portion of the absorbent body and fixing the leakproof wall portions so that the leakproof wall portions are incapable of rising, a back fixing portion being provided in a back end portion of the absorbent body and fixing the leakproof wall portions so that the leakproof wall portions are incapable of rising, an intermediate fixing portion being provided between the front fixing portion and the back fixing portion in the front-back direction, the intermediate fixing portion fixing the leakproof wall portions to the skin-side sheet, the intermediate fixing portion being provided at a position apart from the front fixing portion and the back fixing portion in the front-back direction, the intermediate fixing portion having a first intermediate fixing portion and a second intermediate fixing portion, the first intermediate fixing portion being disposed on a lateral one side, the second intermediate fixing portion being disposed on a lateral other side, a position of a front end of the first intermediate fixing portion and a position of a front end of the second intermediate fixing portion being different in the front-back direction.

According to the above-described absorbent article, a folding guide line that acts as a starting point of the folding of the absorbent body in the front-back direction is not easily formed in the front ends of the first intermediate fixing portion and the second intermediate fixing portion, making it possible to suppress the absorbent body deforming to protrude toward the skin side. Therefore, while the absorbent article is put on, it is possible to suppress the deterioration of the fitting property of the absorbent body in a crotch portion or discomfort caused to wearers.

In such an absorbent article, it is desirable that a position of a back end of the first intermediate fixing portion and a position of a back end of the second intermediate fixing portion are different in the front-back direction.

According to the above-described absorbent article, a folding guide line that acts as a starting point of the folding of the absorbent body in the front-back direction is not easily formed in the back ends of the first intermediate fixing portion and the second intermediate fixing portion, making it possible to suppress the absorbent body deforming to protrude toward the skin side. Therefore, while the absorbent article is put on, it is possible to suppress the deterioration of the fitting property of the absorbent body in a crotch portion or discomfort caused to wearers.

In such an absorbent article, it is desirable that a front-back-direction length of the first intermediate fixing portion and a front-back-direction length of the second intermediate fixing portion are different from each other.

According to the above-described absorbent article, in the first intermediate fixing portion and the second intermediate fixing portion, a straight line connecting their front ends and a straight line connecting their back ends are not parallel to each other. Therefore, in an upper end portion and a lower end portion of the intermediate fixing portion, a force to fold the absorbent body is likely to act in different directions, making it easier to suppress the absorbent body deforming to protrude toward the skin side.

In such an absorbent article, it is desirable that concerning a distance between the front end of the first intermediate fixing portion and the front end of the second intermediate fixing portion in the front-back direction,
the distance is shorter than a front-back-direction length of the first intermediate fixing portion, and
the distance is shorter than a front-back-direction length of the second intermediate fixing portion.

According to the above-described absorbent article, the first intermediate fixing portion and the second intermediate fixing portion have an overlapping portion in the front-back direction, and in the front end, it becomes less likely to form a folding guide line that acts as a starting point of the folding of the absorbent body. This makes it easier for the heights up to which the leakproof wall portions are capable of rising to be equal on the right and left sides. And, this makes it possible to suppress the absorbent body deforming to protrude. Therefore, while the absorbent article is put on, it is easy to suppress the significant deterioration of the fitting property and/or the occurrence of lateral leakage of excrement.

In such an absorbent article, it is desirable that
concerning a distance between a back end of the first intermediate fixing portion and a back end of the second intermediate fixing portion in the front-back direction,
the distance is shorter than a front-back-direction length of the first intermediate fixing portion, and
the distance is shorter than a front-back-direction length of the second intermediate fixing portion.

According to the above-described absorbent article, the first intermediate fixing portion and the second intermediate fixing portion have an overlapping portion in the front-back direction, and in the back end, it becomes less likely to form a folding guide line that acts as a starting point of the folding of the absorbent body. This makes it easier for the heights up to which the leakproof wall portions are capable of rising to be equal on the right and left sides. And, this makes it possible to suppress the absorbent body deforming to protrude. Therefore, while the absorbent article is put on, it is easy to suppress the significant deterioration of the fitting property and/or the occurrence of lateral leakage of excrement.

In such an absorbent article, it is desirable that
the absorbent core has a narrow portion in a central part in the front-back direction,
   the narrow portion having a lateral width smaller than end portions of the absorbent core, and
the front end of the first intermediate fixing portion and the front end of the second intermediate fixing portion overlap the narrow portion in the front-back direction.

According to the above-described absorbent article, even in the case where the absorbent core has a small lateral width and a low rigidity at the positions of the front ends of the first intermediate fixing portion and the second intermediate fixing portion, it is easy to suppress the local folding deformation of the absorbent core occurring at the positions of the front ends. Therefore, it is possible to realize a favorable fitting property in the crotch portion.

In such an absorbent article, it is desirable that
a back end of the first intermediate fixing portion and a back end of the second intermediate fixing portion overlap the narrow portion in the front-back direction.

According to the above-described absorbent article, even in the case where the absorbent core has a small lateral width and a low rigidity at the positions of the back ends of the first intermediate fixing portion and the second intermediate fixing portion, it is easy to suppress the local folding deformation of the absorbent core occurring at the positions of the back ends. Therefore, it is possible to realize a more favorable fitting property in the crotch portion.

In such an absorbent article, it is desirable that
the absorbent core has a narrowest portion in the narrow portion,
   the narrowest portion having a lateral width that is smallest in the narrow portion, and
the first intermediate fixing portion and the second intermediate fixing portion overlap the narrowest portion in the front-back direction.

According to the above-described absorbent article, the first intermediate fixing portion and the second intermediate fixing portion are disposed shifted in the front-back direction, and this makes the local folding deformation of the absorbent core difficult to occur even in the narrowest portion having low rigidity. Therefore, it is possible to suppress the absorbent core deforming to protrude toward the skin side while tightly fitting the absorbent core along the crotch portion of wearers.

In such an absorbent article, it is desirable that
the intermediate fixing portion overlaps at least a part of the stretching/contracting member included in the leakproof wall portion, in a thickness direction.

According to the above-described absorbent article, the stretching/contracting force by the stretching/contracting member provided in the leakproof wall portions is likely to act on the absorbent body through the intermediate fixing portion, making it easier to pull up the absorbent body toward the skin side of wearers in a region where the intermediate fixing portion is disposed. Therefore, it is possible to further enhance the fitting property of the absorbent body in the crotch portion of wearers.

In such an absorbent article, it is desirable that
the intermediate fixing portion is located on a front side in the front-back direction with respect to a center of the absorbent body.

According to the above-described absorbent article, the intermediate fixing portion limits the heights up to which the leakproof wall portions are capable of rising, whereby it is possible to facilitate the planar fitting of the absorbent body to the skin of wearers on the front side. In addition, this makes close the distance between the absorbent body and the excretion opening of wearers, making it possible to facilitate the absorption of excrement such as urine.

In such an absorbent article, it is desirable that
a predetermined space is provided between an outer end of the intermediate fixing portion and an outer end of the leakproof wall portion in the lateral direction.

According to the above-described absorbent article, providing a space at the lateral outer end portion (edge portion) of the leakproof wall portion reduces the hardness of the edge portion, and makes soft the texture of the absorbent article when coming into contact with the body (groin) of wearers while the absorbent article is put on. Therefore, it is possible to realize a more favorable fitting property.

In such an absorbent article, it is desirable that a predetermined space is provided between an inner end of the intermediate fixing portion and an inner end of the leakproof wall portion in the lateral direction.

With the above-described absorbent article, the entire region of the intermediate fixing portion is covered with the leakproof wall portion in the lateral direction, making it less likely to expose an adhesive that forms the intermediate fixing portion to the outside. Therefore, it is possible to suppress the exposed adhesive from adhering to the body or clothing of wearers while the absorbent article is put on.

### Embodiment

Hereinafter, embodiments will be described using an underpants-shaped disposable diaper for adults (hereinafter also referred to as "diaper 1"), as an absorbent article according to the present invention. However, the absorbent article according to the present invention is also available as disposable diapers for infants, sanitary shorts, and the like.

### Configuration of diaper 1

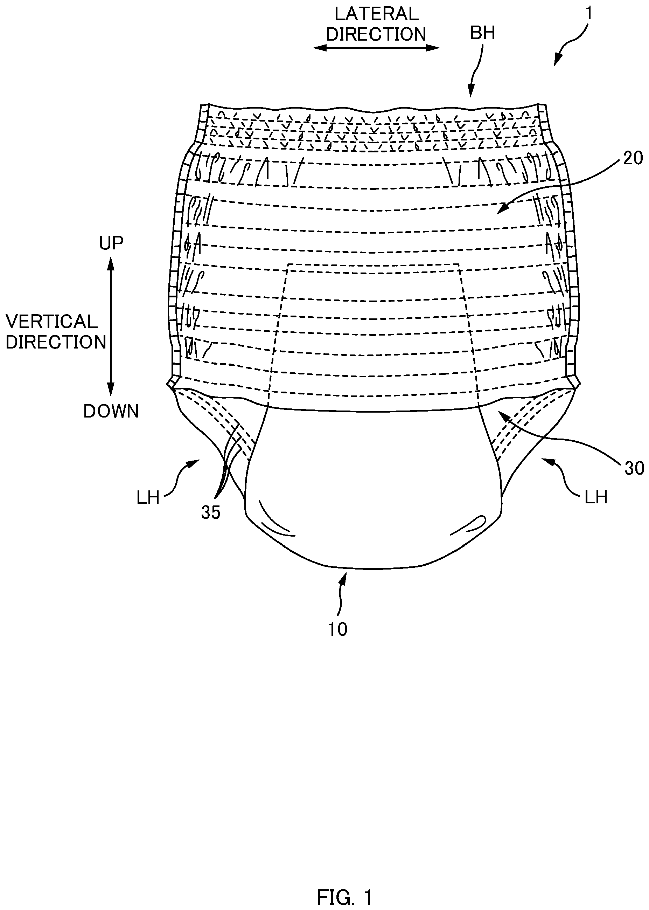

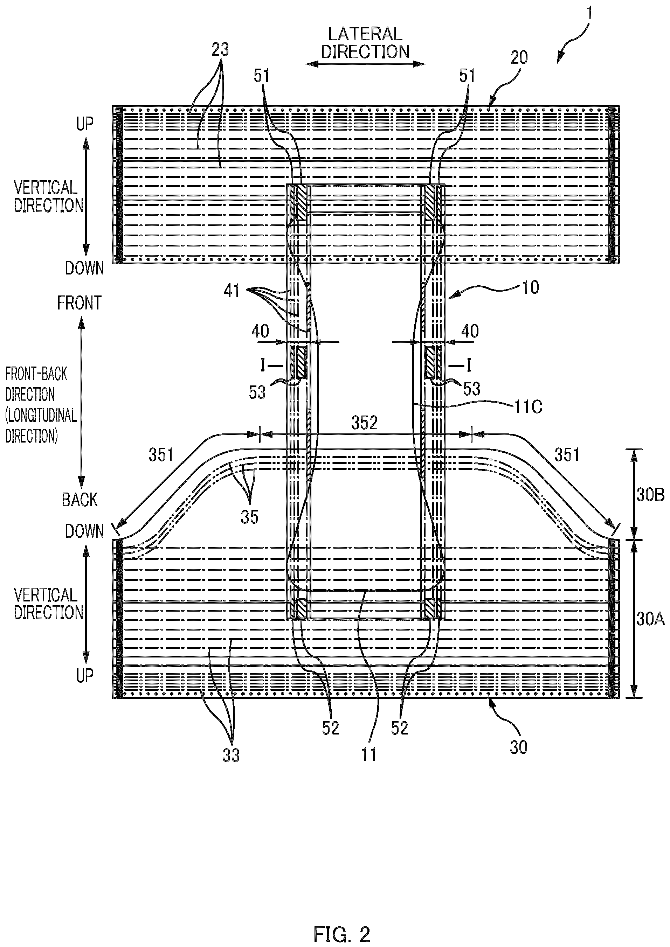

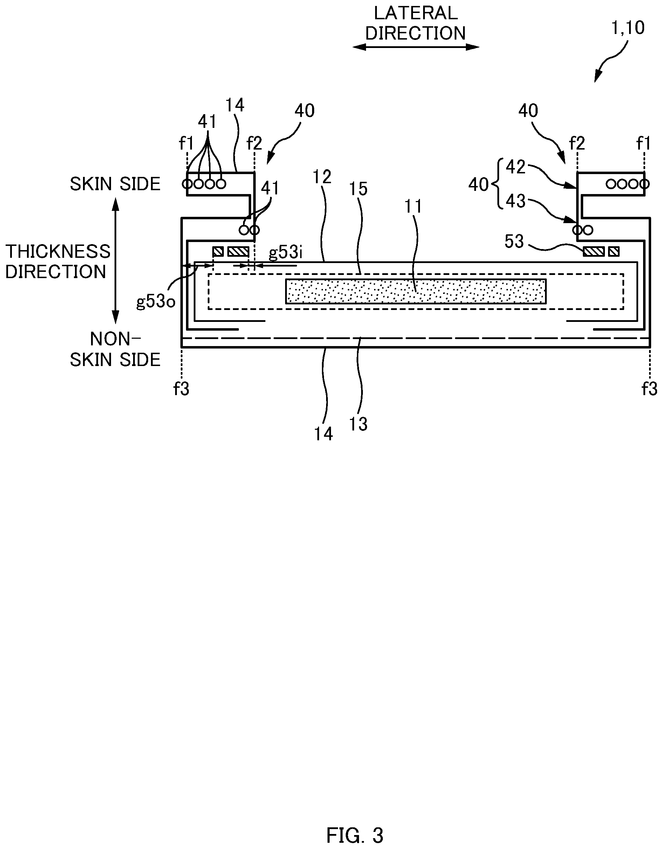

FIG. 1 is a schematic front view of the diaper 1. FIG. 2 is a schematic plan view of the diaper 1 in a stretched state. FIG. 3 is a schematic cross-sectional view taken along a line I-I in FIG. 2. It should be noted that the "stretched state" of the diaper 1 refers to a state in which the entire diaper 1 (the entire product) is stretched without forming wrinkles, specifically, a state in which the diaper is stretched such that the dimensions of constituent members of the diaper 1 (e.g., an absorbent body 10, waist portions 20 and 30, or the like that will be described later) match or are close to the dimensions of the members on their own.

In an underpants-shaped state shown in FIG. 1, the diaper 1 has a vertical direction, a lateral direction, and a front-back direction that intersect each other and has a waist opening BH and a pair of leg openings LH. The upper side in the vertical direction corresponds to the waist opening BH side, and the lower side corresponds to the crotch side. In addition, the front side in the front-back direction corresponds to the front side of a wearer, and the back side corresponds to the back side of the wearer. In addition, in an unfolded state of FIG. 2, the diaper 1 has the front-back direction and the lateral direction that intersect each other. The front-back direction corresponds to the longitudinal direction of the absorbent body 10. In addition, as shown in FIG. 3, a direction in which constituent members of the diaper 1 are stacked is referred to as a thickness direction. In the thickness direction, a side that comes into contact with the wearer is defined as a skin side, and a side opposite to the skin side is defined as a non-skin side.

The diaper 1 has an absorbent body 10, a front waist portion 20, and a back waist portion 30. In the diaper 1 in the underpants-shaped state, the absorbent body 10 (an absorbent core 11 described below) is disposed extending along the vertical direction and is folded in the front-back direction, at the lower end portion in the vertical direction. In addition, the front waist portion 20 is joined to the non-skin side of the front upper end portion of the absorbent body 10, and the back waist portion 30 is joined to the non-skin side of the back upper end portion of the absorbent body 10.

In the diaper 1 in the unfolded state of FIG. 2, the front waist portion 20 and the back waist portion 30 are disposed such that the longitudinal directions thereof are along the lateral direction of the diaper 1. In addition, the longitudinal one-side (front) end portion of the absorbent body 10 is disposed in the lateral central part of the front waist portion 20, and the longitudinal other-side (back) end portion of the absorbent body 10 is disposed in the lateral central part of the back waist portion 30. In the unfolded state shown in FIG. 2, the absorbent body 10 is folded one time at substantially the center in the front-back direction (longitudinal direction), and two lateral side portions of the front waist portion 20 and two lateral side portions of the back waist portion 30 are respectively joined by welding or the like, thereby forming the diaper 1 in the underpants-shaped state.

The absorbent body 10 has the absorbent core 11 that absorbs excrement, a hydrophilic skin-side sheet 12 disposed on the wearer's skin side of the absorbent core 11, a liquid-impermeable non-skin-side sheet 13 disposed on the non-skin side of the absorbent core 11, and a sheet member 14. Two lateral side portions of the skin-side sheet 12 are folded back toward the non-skin side so as to enclose the absorbent core 11. The sheet member 14 is disposed on the non-skin side with respect to the non-skin-side sheet 13 and forms leakproof wall portions 40 on the skin side with respect to the skin-side sheet 12.

The absorbent core 11 is an absorbent core obtained by shaping a liquid absorbent fiber such as a pulp fiber containing a superabsorbent polymer (SAP) into a predetermined shape. In addition, the absorbent core 11 may be covered with a liquid-permeable core wrap sheet 15.

In addition, the absorbent core 11 of the present embodiment has a narrow portion 11C in the central part in the front-back direction, and the narrow portion 11C is a portion where the lateral length of the absorbent core 11 is shorter (has a narrower width) compared with the front end portion and the back end portion. Accordingly, the absorbent core 11 has a substantially hourglass shape in a plan view as shown in FIG. 2. This narrow portion 11C is a portion sandwiched between both legs of the wearer while the diaper 1 is put on, and the lateral length thereof is small (the width is narrow), making the absorbent core 11 easier to fit to the crotch of the wearer.

The front waist portion 20 (the back waist portion 30) includes an inner-layer sheet 21 (31), an outer-layer sheet 22 (32) disposed on the non-skin side with respect to the inner-layer sheet 21 (31), and waist elastic members 23 (33). The waist elastic members 23 (33) are disposed side by side in the vertical direction between the inner-layer sheet 21 (31) and the outer-layer sheet 22 (32) and are fixed in a laterally stretched state. Therefore, the front waist portion 20 and the back waist portion 30 fit the waist of the wearer. In addition, a cover sheet (not shown) that covers the upper end portion of the absorbent body 10 from the skin side may also be provided.

In the unfolded and stretched state of FIG. 2, the front waist portion 20 has a rectangular shape in a plan view. The back waist portion 30 includes: an overlapping portion 30A that overlaps the front waist portion 20 in the front-back direction; and an extension portion 30B that extends downward beyond the front waist portion 20. The overlapping portion 30A has a rectangular shape in a plan view, and the extension portion 30B has an inverted trapezoidal shape in a plan view. The buttocks of the wearer can be covered with the extension portion 30B. The front waist portion 20 has a short vertical length compared with the back waist portion 30 and does not extend up to the crotch side. Therefore, the movement of legs that are pulled up to the front side is not hindered during walking or the like, and the wearer easily walks.

In addition, in the extension portion 30B of the back waist portion 30, extension-portion elastic members 35 are provided along the lower end of the extension portion 30B. Specifically, the extension-portion elastic member 35 has: a pair of inclined portions 351 extending downward and inclined inward in the lateral direction; and a straight portion 352 extending along the lateral direction and between the pair of inclined portions 351. Similar to the waist elastic members 33, the extension-portion elastic members 35 are fixed in a stretched state, between the inner-layer sheet 31 and the outer-layer sheet 32. The extension-portion elastic members 35 fit the extension portion 30B to the buttocks of the wearer, making it possible to suppress the extension portion 30B being curling-up.

However, the configuration is not limited thereto. For example, the front waist portion 20 may have the same planar shape as the back waist portion 30 and may include the same elastic members as the extension-portion elastic members 35 in the back waist portion 30. That is, the front waist portion 20 may include inclined elastic members extending downward and inclined inward in the lateral direction, at the lower end portion. In addition, conversely, the back waist portion 30 may have the same rectangular shape in a plan view as the front waist portion 20 and may not include the extension-portion elastic members 35.

On two lateral side portions of the absorbent body 10, a pair of the leakproof wall portions 40 are respectively provided extending along the front-back direction (the longitudinal direction of the absorbent body 10). In the present embodiment, the pair of leakproof wall portions 40 is respectively formed by folding the sheet member 14 that constitutes the exterior of the absorbent body 10, at multiple positions as shown in FIG. 3. Specifically, the sheet member 14 is folded at folding positions f1 in FIG. 3 into two layers, and is further folded at folding positions f2 and folding positions f3. Thus, the leakproof wall portions 40 are formed being folded so as to have a substantially S-shaped cross-section.

In the leakproof wall portion 40, a portion between the folding lines f1 and f2 is defined as a "skin-side portion 42", and a portion between the folding lines f2 and f3, which is disposed on the non-skin side of the skin-side portion 42, is defined as a "non-skin-side portion 43". The skin-side portion 42 is a portion folded outward in the lateral direction relative to the non-skin-side portion 43.

In addition, in the leakproof wall portion 40, between the two overlaid layers of the sheet member 14, a predetermined stretching/contracting member is fixed in a state of being stretched in the front-back direction of the absorbent body 10 (corresponding to the vertical direction of the diaper 1). In the present embodiment, as the stretching/contracting member, a plurality of leakproof-wall stretching/contracting members 41 such as elastic strings are disposed side by side in the lateral direction. While the diaper 1 is put on, the stretchability generated by the leakproof-wall stretching/contracting members 41 (stretching/contracting member) makes the leakproof wall portions 40 contract along the front-back direction of the absorbent body 10 and rise toward the skin side of the wearer, fitting the diaper 1 to the crotch portion of the wearer.

It should be noted that the leakproof wall portions 40 are fixed by front fixing portions 51 provided at the front end portion, so as to be incapable of rising in the front end portion of the absorbent body 10 in the front-back direction. Similarly, the leakproof wall portions 40 are fixed by back fixing portions 52 provided at the back end portion, so as to be incapable of rising in the back end portion of the absorbent body 10 in the front-back direction (see FIG.2).

Therefore, the leakproof wall portions 40 become capable of rising toward the skin side of the wearer, in a region between the front fixing portions 51 and the back fixing portions 52 in the front-back direction of the absorbent body 10. In addition, for example, in a case where the wearer is in a lying-down posture, even when liquid excrement held by the leakproof wall portions 40 flows in the front-back direction, it is possible for the front fixing portions 51 and the back fixing portions 52 to block the liquid excrement, making it possible to suppress front leakage or back leakage.

In addition, between the front fixing portions 51 and the back fixing portions 52 in the front-back direction of the absorbent body 10, intermediate fixing portions 53 that fix the leakproof wall portions 40 to the skin-side sheet 12 of the absorbent body 10 are provided. As shown in FIG. 2, the intermediate fixing portions 53 of the present embodiment are provided apart from the front fixing portions 51 and the back fixing portions 52 in the front-back direction. That is, in the absorbent body 10, the front fixing portions 51, the intermediate fixing portions 53, and the back fixing portions 52 are intermittently disposed in the front-back direction in a state in which the diaper 1 is unfolded and stretched. It should be noted that all of the front fixing portions 51, the back fixing portions 52, and the intermediate fixing portions 53 are formed of an adhesive such as a hot melt adhesive.

In portions in the front-back direction where the intermediate fixing portions 53 are provided, since the non-skin-side portions 43 of the leakproof wall portions 40 are fixed to the skin-side sheet 12 (see FIG. 3), only the skin-side portions 42 are capable of rising toward the skin side. That is, in the portions where the intermediate fixing portions 53 are provided, the height up to which the leakproof wall portions 40 are capable of rising is limited, and it is possible to decrease the gap between the absorbent body 10 and the skin of the wearer while the diaper 1 is put on. Accordingly, appropriately adjusting the disposition or size of the intermediate fixing portions 53 makes it possible to change the fitting property of the diaper 1 around the wearer's legs and/or the excrement absorption performance.

### Disposition of intermediate fixing portions 53

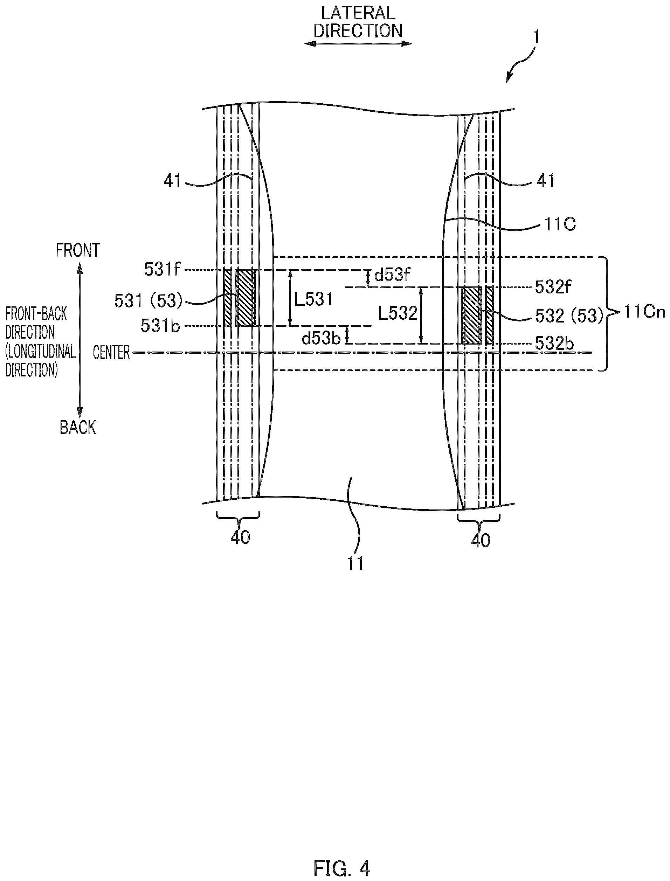

FIG. 4 is an enlarged plan view illustrating the disposition of the intermediate fixing portions 53. FIG. 4 shows, similar to FIG. 2, the absorbent body 10 in an unfolded and stretched state. In addition, of the pair of intermediate fixing portions 53 and 53 provided on the right and left sides of the absorbent body 10, the intermediate fixing portion disposed on one side in the lateral direction (on the left side in FIG. 4) is defined as a first intermediate fixing portion 531, and the intermediate fixing portion disposed on the other side in the lateral direction (on the right side in FIG. 4) is defined as a second intermediate fixing portion 532.

In the diaper 1, the position of a front end 531f of the first intermediate fixing portion 531 and the position of a front end 532f of the second intermediate fixing portion 532 are different in the front-back direction. That is, the front end 531f and the front end 532f are not arranged in the same straight line extending in the lateral direction. Similarly, the position of a back end 531b of the first intermediate fixing portion 531 and the position of a back end 532b of the second intermediate fixing portion 532 are different in the front-back direction. With such a configuration, it is possible to suppress the absorbent body 10 being folded in a region where the intermediate fixing portions 53 are provided.

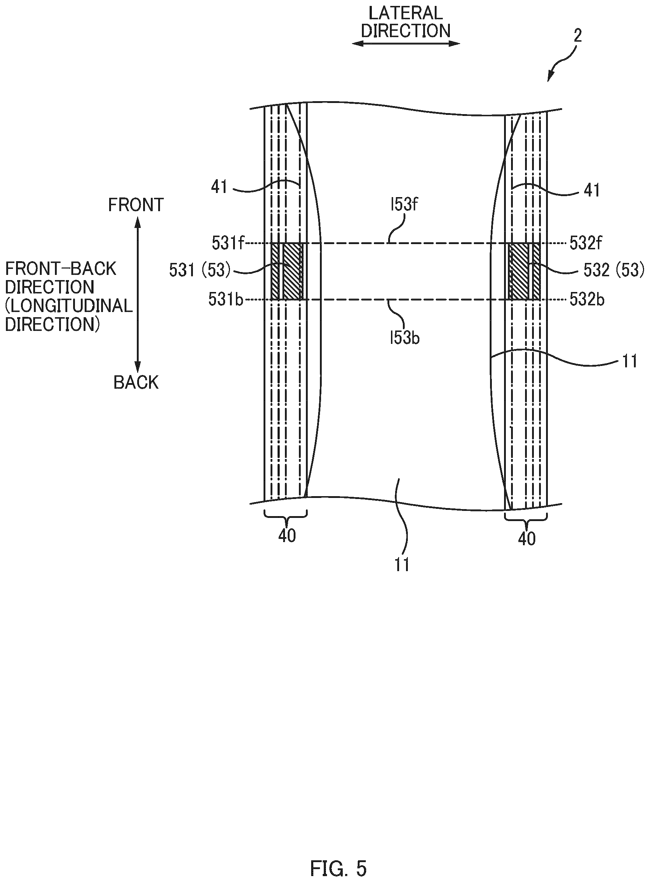

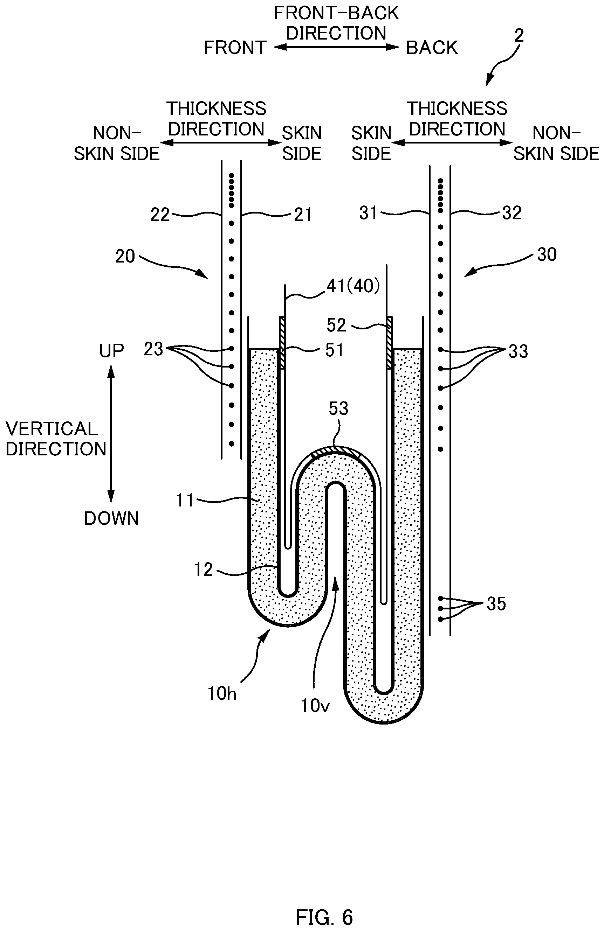

The folding of the absorbent body 10 will be described using a diaper 2 as a comparative example. FIG. 5 is an enlarged plan view illustrating the disposition of the intermediate fixing portions 53 in the diaper 2 of the comparative example. FIG. 6 is a schematic cross-sectional view of the diaper 2 in an underpants-shaped state, cut in the front-back direction. In the diaper 2 of the comparative example, the first intermediate fixing portion 531 and the second intermediate fixing portion 532 are provided symmetrically in the lateral direction. That is, the first intermediate fixing portion 531 and the second intermediate fixing portion 532 have the same shape (size) and are disposed at the same position in the front-back direction. Therefore, as shown in FIG. 5, the front end 531f and the front end 532f are disposed side by side on the same straight line extending in the lateral direction, and the back end 531b and the back end 532b are disposed side by side on the same straight line extending in the lateral direction. It should be noted that the diaper 1 of the present embodiment and the diaper 2 of the comparative example have almost the same configuration except that the disposition of the first intermediate fixing portion 531 and the second intermediate fixing portion 532 is different.

When the diaper 2 in an unfolded state is made into an underpants-shaped state, the leakproof-wall stretching/contracting members 41 (stretching/contracting member) contract along the front-back direction (the longitudinal direction of the absorbent body 10). In addition, in the portions where the intermediate fixing portions 53 are provided, the leakproof wall portions 40 (the non-skin-side portions 43) and the absorbent body 10 (the skin-side sheet 12) are joined to each other, and therefore the absorbent body 10 is pulled toward the skin side of the wearer due to the contraction of the leakproof-wall stretching/contracting members 41 (the stretching/contracting member). At this time, the absorbent body 10 is likely to fold in the front-back direction at the ends of the intermediate fixing portions 53 in the front-back direction (for example, the front end 531f). This is because, due to the adhesive applied to the intermediate fixing portions 53, the intermediate fixing portion 53 has higher rigidity than other regions, the difference in rigidity becomes large at the end edges of the intermediate fixing portions 53, and thus it is likely that stress concentrates and a folding starting point is formed.

As shown in FIG. 5, in the diaper 2, the front end 531f and the front end 532f are disposed at the same position in the front-back direction, and act as folding starting points. Therefore, concerning a straight line I53f which connects the front end 531f and the front end 532f and extends along the lateral direction, the straight line I53f works as a folding starting point (folding guide line), and the absorbent body 10 is likely to be folded along the straight line I53f. Similarly, the back end 531b and the back end 532b are disposed at the same position in the front-back direction. And concerning a straight line I53b which connects the back end 531b and the back end 532b and which extends along the lateral direction, the straight line I53b works as a folding starting point, and the absorbent body 10 is likely to be folded along the straight line I53b.

As a result, the region in the absorbent body 10 where the intermediate fixing portions 53 are provided deforms into a shape protruding toward the skin side as shown in FIG. 6, thereby forming a recessed portion 10v. In addition, the formation of the recessed portion 10v forms a protruding portion 10h in front of the recessed portion 10v. When a recess and a protrusion such as the recessed portion 10v and the protruding portion 10h are formed in the absorbent body 10, the fitting property of the absorbent body 10 in the crotch portion deteriorates while the diaper 2 is put on. In addition, the recessed portion 10v and the protruding portion 10h are formed adjacent to each other in the front-back direction, and this makes the absorbent body 10 (particularly, the absorbent core 11) be in a state of being folded and overlapped in the crotch portion, making it more likely to cause discomfort to the wearer.

In contrast, in the diaper 1 of the present embodiment, as shown in FIG. 4, the position of the front end 531f of the first intermediate fixing portion 531 and the position of the front end 532f of the second intermediate fixing portion 532 are different in the front-back direction. This make it difficult to form a folding guide line that extends along the lateral direction as in the comparative example (the straight line 153f in FIG. 5), in the front ends of the first intermediate fixing portion 531 and the second intermediate fixing portion 532. And, it is easy to suppress the absorbent body 10 deforming to protrude toward the skin side. Accordingly, while the diaper 1 is put on, it is possible to suppress the deterioration of the fitting property of the absorbent body 10 in the crotch portion or discomfort caused to the wearer.

Similarly, the position of the back end 531b of the first intermediate fixing portion 531 and the position of the back end 532b of the second intermediate fixing portion 532 are different in the front-back direction. This make it difficult to form a folding guide line that extends along the lateral direction (the straight line l53b in FIG. 5), in the back ends of the first intermediate fixing portion 531 and the second intermediate fixing portion 532. And, it is easy to suppress the absorbent body 10 deforming to protrude toward the skin side. Accordingly, while the diaper 1 is put on, it is possible to suppress the deterioration of the fitting property of the absorbent body 10 in the crotch portion or discomfort caused to the wearer.

In addition, in the diaper 1, the distance L531 in the front-back direction between the front end 531f and the back end 531b of the first intermediate fixing portion 531 is different from the distance L532 in the front-back direction between the front end 532f and the back end 532b of the second intermediate fixing portion 532. That is, the front-back-direction length L531 of the first intermediate fixing portion 531 is different from the front-back-direction length L532 of the second intermediate fixing portion. As described above, in a case where the lengths of the pair of intermediate fixing portions 531 and 532 disposed on the right and left sides are different from each other, a straight line connecting the front end 531f and the front end 532f and a straight line connecting the back end 531b and the back end 532b are not parallel to each other. Therefore, in the upper end portions and the lower end portions of the intermediate fixing portions 53, a force to fold the absorbent body 10 is likely to act in different directions, suppressing the absorbent body 10 deforming to protrude toward the skin side. This makes it difficult to form the recessed portion 10v as shown in FIG. 6, making it easier to suppress the deterioration of the fitting property.

In addition, as shown in FIG. 4, in the front-back direction, the distance d53f between the front end 531f of the first intermediate fixing portion 531 and the front end 532f of the second intermediate fixing portion 532 is shorter than the front-back-direction length L531 of the first intermediate fixing portion 531 and the front-back-direction length L532 of the second intermediate fixing portion 532 (d53f < L531 and L532). That is, the first intermediate fixing portion 531 and the second intermediate fixing portion 532 have an overlapping portion in the front-back direction and are disposed such that the front ends are shifted by the distance d53f.

As described above, in the region where the intermediate fixing portions 53 are provided, the heights up to which the leakproof wall portions 40 are capable of rising are limited, making it possible to adjust the fitting property while the diaper 1 is put on. In a case where the shift amount d53f for the front ends of the first intermediate fixing portion 531 and the second intermediate fixing portion 532 is larger than the length L531 of the first intermediate fixing portion 531 (or the length L532 of the second intermediate fixing portion 532), the first intermediate fixing portion 531 and the second intermediate fixing portion 532 do not have any overlapping portion in the front-back direction. In this case, on the right and left sides, different are regions where the heights up to which the leakproof wall portions 40 are capable of rising are limited. And there are a risk that, while the diaper 1 is put on, the fitting property significantly deteriorates and/or the lateral leakage of excrement occurs.

In contrast, the diaper 1 of the present embodiment has at least a portion where the first intermediate fixing portion 531 and the second intermediate fixing portion 532 overlap in the front-back direction. This makes it easier for the heights up to which the leakproof wall portions 40 are capable of rising to be equal on the right and left sides. And, while the diaper 1 is put on, the significant deterioration of the fitting property and/or the occurrence of lateral leakage of excrement is suppressed.

Similarly, in the front-back direction, the distance d53b between the back end 531b of the first intermediate fixing portion 531 and the back end 532b of the second intermediate fixing portion 532 is shorter than the front-back-direction length L531 of the first intermediate fixing portion 531 and the front-back-direction length L532 of the second intermediate fixing portion 532 (d53b < L531 and L532). That is, the first intermediate fixing portion 531 and the second intermediate fixing portion 532 have an overlapping portion in the front-back direction and are disposed such that the back ends are shifted by the distance d53b. Therefore, while the diaper 1 is put on, the significant deterioration of the fitting property and/or the occurrence of lateral leakage of excrement is suppressed.

In addition, in the diaper 1, the front end 531f of the first intermediate fixing portion 531 and the front end 532f of the second intermediate fixing portion 532 overlap the narrow portion 11C of the absorbent core 11 in the front-back direction. That is, the intermediate fixing portions 53 have a portion that overlaps the portion where the lateral length (width) of the absorbent core 11 is narrow, in the front-back direction. As described above, providing the narrow portion 11C can make it easier to fit the absorbent core 11 to the crotch portion of the wearer while the diaper 1 is put on. On the other hand, since the narrow portion 11C has a small lateral width, the rigidity is low compared with other regions (for example, the front and back end portions of the absorbent core 11), making it likely for the absorbent core 11 to fold and deform. Accordingly, there is a risk of forming the recessed portion 10v as shown in FIG. 6.

In contrast, in the diaper 1 of the present embodiment, the positions of the front end 531f of the first intermediate fixing portion 531 and the front end 532f of the second intermediate fixing portion 532 are shifted in the front-back direction, and therefore a folding starting point of the absorbent body 10 is less likely to be formed at the front ends 531f and 532f. Therefore, even in the case where the absorbent core 11 has a small width and a low rigidity at the positions of the front ends 531f and 532f, the local folding deformation of the absorbent core 11 is suppressed, making it less likely to form the recessed portion 10v. Therefore, it is possible to realize a favorable fitting property in the crotch portion.

Similarly, in the diaper 1, the back end 531b of the first intermediate fixing portion 531 and the back end 532b of the second intermediate fixing portion 532 overlap the narrow portion 11C of the absorbent core 11 in the front-back direction. Therefore, even in the case where the absorbent core 11 has a small width and a low rigidity at the positions of the back ends 531b and 532b, the local folding deformation of the absorbent core 11 is suppressed, making it less likely to form the recessed portion 10v.

It should be noted that the absorbent core 11 has a narrowest portion 11Cn that is a region where the width in the lateral direction is narrowest in the narrow portion 11C. In addition, both the first intermediate fixing portion 531 and the second intermediate fixing portion 532 of the diaper 1 are disposed at positions that overlap the narrowest portion 11Cn in the front-back direction. In the narrowest portion 11Cn, the rigidity of the absorbent core 11 is the lowest, and folding deformation is likely to occur. However, in the present embodiment, the first intermediate fixing portion 531 and the second intermediate fixing portion 532 are shifted in the front-back direction as described above, and this makes local folding deformation difficult to occur. Therefore, it is possible to suppress the absorbent core deforming to protrude toward the skin side, like the recessed portion 10v, while tightly fitting the absorbent core 11 along the crotch portion of the wearer.

In addition, the first intermediate fixing portion 531 and the second intermediate fixing portion 532 overlap at least a part of the leakproof-wall stretching/contracting members 41 in the thickness direction. In FIG. 3, the first intermediate fixing portion 531 and the second intermediate fixing portion 532 are disposed at positions that overlap the leakproof-wall stretching/contracting members 41 provided in the non-skin-side portions 43 of the leakproof wall portions 40, in the thickness direction. Therefore, the stretching/contracting force by the leakproof-wall stretching/contracting members 41 is likely to act on the absorbent body 10 through the intermediate fixing portions 53, making it easier to pull up the absorbent body 10 toward the skin side of the wearer in the regions where the intermediate fixing portions 53 are disposed. Therefore, it is possible to further enhance the fitting property of the absorbent body 10 in the crotch portion of the wearer.

Note that it is desirable that the front end 531f of the first intermediate fixing portion 531 and the front end 532f of the second intermediate fixing portion 532 are located on the front side in the front-back direction with respect to the center of the absorbent body 10. In the body of the wearer, a protrusion and a recess are smaller in a region on the stomach side than on the buttocks side. Accordingly, when the intermediate fixing portions 53 restricts the possible rising heights of the leakproof wall portions 40, this can make it easier to fit the absorbent body 10 in a planar manner to the skin (front side) of the wearer. In addition, this makes close the distance between the absorbent body 10 and the excretion opening of the wearer, making it possible to facilitate the absorption of excrement such as urine.

Furthermore, it is desirable that the back end 531b of the first intermediate fixing portion 531 and the back end 532b of the second intermediate fixing portion 532 are located on the front side in the front-back direction with respect to the center of the absorbent body 10. With such a configuration, all of the intermediate fixing portions 53 are disposed in a front region in the front-back direction, and this can make it easier to further fit the absorbent body 10 to the skin (front side) of the wearer on the front side of the wearer. In addition, it is possible to further facilitate the absorption of excrement such as urine.

In addition, the intermediate fixing portions 53 are disposed at positions apart from the outer ends (the folding positions f3 in FIG. 3) of the leakproof wall portions 40 by a predetermined distance g53o in the lateral direction. In other words, between the intermediate fixing portion 53 and the outer end of the leakproof wall portion 40 in the lateral direction, a predetermined space (g53o) is provided. The provision of such spaces reduces the hardness of the lateral outer ends (edges) of the leakproof wall portions 40 and makes soft the texture of the diaper 1 when coming into contact with the body (groin) of the wearer while the diaper 1 is put on. Therefore, it is possible to realize a more favorable fitting property.

In addition, the intermediate fixing portions 53 are disposed at positions apart from the inner ends (the folding positions f2 in FIG. 3) of the leakproof wall portions 40 by a predetermined distance g53i in the lateral direction. In other words, between the intermediate fixing portion 53 and the inner end of the leakproof wall portion 40 in the lateral direction, a space (g53i) is provided. Therefore, the entire regions of the intermediate fixing portions 53 are covered with the leakproof wall portions 40 (non-skin-side portions 43) from the skin side, making it less likely to expose the adhesive that forms the intermediate fixing portions 53 outside. This makes it possible to suppress the exposed adhesive from adhering to the body or clothing of wearers while the diaper 1 is put on.

### Other embodiments

Hitherto, the embodiment of the present invention has been described, but the above-described embodiment is intended to facilitate the understanding of the present invention and is not intended to limit the interpretation of the present invention. In addition, the present invention can be modified or improved without departing from the spirit thereof, and additionally, it is needless to say that equivalents thereof are included in the present invention.

In the above-described embodiment, a diaper in which the front waist portion 20 and the back waist portion 30 are separate members has been exemplified. However, the front waist portion 20 and the back waist portion 30 may be a single member that is continuous by a crotch portion provided between the front waist portion 20 and the back waist portion 30.

In the above-described embodiment, the leakproof wall portions 40 are formed by folding the sheet member 14. That is, the leakproof wall portions 40 are formed in an integrated manner with the sheet member 14. However, the present invention is not limited thereto. For example, the leakproof wall portions 40 may be formed of a separate sheet member that is different from the sheet member 14.

### [Reference Signs List]

1 diaper (absorbent article),
2 diaper (comparative example),
10 absorbent body, 11 absorbent core, 11C narrow portion, 11Cn narrowest portion,
10h protruding portion, 10v recessed portion,
12 skin-side sheet, 13 non-skin-side sheet,
14 sheet member, 15 core wrap sheet,
20 front waist portion, 21 inner-layer sheet, 22 outer-layer sheet,
23 waist elastic member,
30 back waist portion, 31 inner-layer sheet, 32 outer-layer sheet,
33 waist elastic member,
35 extension-portion elastic member, 351 inclined portion, 352 straight portion,
40 leakproof wall portion,
41 leakproof-wall stretching/contracting member (stretching/contracting member), 42 skin-side portion, 43 non-skin-side portion,
51 front fixing portion, 52 back fixing portion,
53 intermediate fixing portion,
531 first intermediate fixing portion, 531f front-side end, 531b back-side end,
532 second intermediate fixing portion, 532f front-side end, 532b back-side end,
BH waist opening, LH leg opening

## Claims

1. An absorbent article (1) comprising:
an absorbent body (10) including an absorbent core (11) and a skin-side sheet (12) disposed on a skin side with respect to the absorbent core (11); and
a pair of leakproof wall portions (40) including a stretching/contracting member (41) that stretches and contracts,
in a state in which the absorbent article (1) is unfolded, the absorbent body (10) having a front-back direction and a lateral direction that intersect each other,
the pair of leakproof wall portions (40) being provided on two lateral sides of the absorbent body (10),
a front fixing portion (51) being provided in a front end portion of the absorbent body (10) and fixing the leakproof wall portions (40) so that the leakproof wall portions (40) are incapable of rising,
a back fixing portion (52) being provided in a back end portion of the absorbent body (10) and fixing the leakproof wall portions (40) so that the leakproof wall portions (40) are incapable of rising,
an intermediate fixing portion (53) being provided between the front fixing portion (51) and the back fixing portion (52) in the front-back direction,
the intermediate fixing portion (53) fixing the leakproof wall portions (40) to the skin-side sheet (12),
the intermediate fixing portion (53) being provided at a position apart from the front fixing portion (51) and the back fixing portion (52) in the front-back direction,
the intermediate fixing portion (53) having a first intermediate fixing portion (531) and a second intermediate fixing portion (532),
the first intermediate fixing portion (531) being disposed on a lateral one side,
the second intermediate fixing portion (532) being disposed on a lateral other side,
a position of a front end (531f) of the first intermediate fixing portion (531) and a position of a front end (532f) of the second intermediate fixing portion (532) being different in the front-back direction.

2. The absorbent article (1) according to claim 1, wherein
a position of a back end (531b) of the first intermediate fixing portion (531) and a position of a back end (532b) of the second intermediate fixing portion (532) are different in the front-back direction.

3. The absorbent article (1) according to claim 1 or 2, wherein
a front-back-direction length (L531) of the first intermediate fixing portion (531) and a front-back-direction length (L532) of the second intermediate fixing portion (532) are different from each other.

4. The absorbent article (1) according to any one of claims 1 to 3, wherein
concerning a distance (d53f) between the front end (531f) of the first intermediate fixing portion (531) and the front end (532f) of the second intermediate fixing portion (532) in the front-back direction,
the distance (d53f) is shorter than a front-back-direction length (L531) of the first intermediate fixing portion (531), and
the distance (d53f) is shorter than a front-back-direction length (L532) of the second intermediate fixing portion (532).

5. The absorbent article (1) according to any one of claims 1 to 4, wherein
concerning a distance (d53b) between a back end (531b) of the first intermediate fixing portion (531) and a back end (532b) of the second intermediate fixing portion (532) in the front-back direction,
the distance (d53b) is shorter than a front-back-direction length (L531) of the first intermediate fixing portion (531), and
the distance (d53b) is shorter than a front-back-direction length (L532) of the second intermediate fixing portion (532).

6. The absorbent article (1) according to any one of claims 1 to 5, wherein
the absorbent core (11) has a narrow portion (11C) in a central part in the front-back direction,
the narrow portion (11C) having a lateral width smaller than end portions of the absorbent core (11), and
the front end (531f) of the first intermediate fixing portion (531) and the front end (532f) of the second intermediate fixing portion (532) overlap the narrow portion (11C) in the front-back direction.

7. The absorbent article (1) according to claim 6, wherein
a back end (531b) of the first intermediate fixing portion (531) and a back end (532b) of the second intermediate fixing portion (532) overlap the narrow portion (11C) in the front-back direction.

8. The absorbent article (1) according to claim 6 or 7, wherein
the absorbent core (11) has a narrowest portion (11Cn) in the narrow portion (11C),
the narrowest portion (11Cn) having a lateral width that is smallest in the narrow portion (11C), and
the first intermediate fixing portion (531) and the second intermediate fixing portion (532) overlap the narrowest portion (11Cn) in the front-back direction.

9. The absorbent article (1) according to any one of claims 1 to 8, wherein
the intermediate fixing portion (53) overlaps at least a part of the stretching/contracting member (41) included in the leakproof wall portion (40), in a thickness direction.

10. The absorbent article (1) according to any one of claims 1 to 9, wherein
the intermediate fixing portion (53) is located on a front side in the front-back direction with respect to a center of the absorbent body (10).

11. The absorbent article (1) according to any one of claims 1 to 10, wherein
a predetermined space (g53o) is provided between an outer end of the intermediate fixing portion (53) and an outer end of the leakproof wall portion (40) in the lateral direction.

12. The absorbent article (1) according to any one of claims 1 to 11, wherein
a predetermined space (g53i) is provided between an inner end of the intermediate fixing portion (53) and an inner end of the leakproof wall portion (40) in the lateral direction.

## Patentansprüche

1. Absorbierender Artikel (1), der Folgendes umfasst:
einen Saugkörper (10), der einen Saugkern (11) und eine Hautseitenlage (12), die auf einer Hautseite in Bezug auf den Saugkern (11) angeordnet ist, einschließt; und
ein Paar von auslaufsicheren Wandteilen (40), die ein sich dehnendes/zusammenziehendes Element (41) einschließen, das sich dehnt und zusammenzieht,
wobei in einem Zustand, in dem der absorbierende Artikel (1) entfaltet ist, der Saugkörper (10) eine Vorn-Hinten-Richtung und eine laterale Richtung aufweist, die einander schneiden,
wobei das Paar von auslaufsicheren Wandteilen (40) auf zwei lateralen Seiten des Saugkörpers (10) bereitgestellt ist,
einen vorderen fixierenden Teil (51), der in einem vorderen Endteil des Saugkörpers (10) bereitgestellt ist und die auslaufsicheren Wandteile (40) fixiert, sodass sich die auslaufsicheren Wandteile (40) nicht aufstellen können,
einen hinteren fixierenden Teil (52), der in einem hinteren Endteil des Saugkörpers (10) bereitgestellt ist und die auslaufsicheren Wandteile (40) fixiert, sodass sich die auslaufsicheren Wandteile (40) nicht aufstellen können,
einen dazwischenliegenden fixierenden Teil (53), der zwischen dem vorderen fixierenden Teil (51) und dem hinteren fixierenden Teil (52) in der Vorn-Hinten-Richtung bereitgestellt ist,
wobei der dazwischenliegende fixierende Teil (53) die auslaufsicheren Wandteile (40) an der Hautseitenlage (12) fixiert,
wobei der dazwischenliegende fixierende Teil (53) an einer Position bereitgestellt ist, die von dem vorderen fixierenden Teil (51) und dem hinteren fixierenden Teil (52) in der Vorn-Hinten-Richtung entfernt ist,
der dazwischenliegende fixierende Teil (53) einen ersten dazwischenliegenden fixierenden Teil (531) und einen zweiten dazwischenliegenden fixierenden Teil (532) aufweist,
wobei der erste dazwischenliegende fixierende Teil (531) auf einer lateralen Seite angeordnet ist,
der zweite dazwischenliegende fixierende Teil (532) auf einer anderen lateralen Seite angeordnet ist,
wobei eine Position eines vorderen Endes (531f) des ersten dazwischenliegenden fixierenden Teils (531) und eine Position eines vorderen Endes (532f) des zweiten dazwischenliegenden fixierenden Teils (532) in der Vorn-Hinten-Richtung verschieden sind.

2. Absorbierender Artikel (1) nach Anspruch 1, wobei
eine Position eines hinteren Endes (531b) des ersten dazwischenliegenden fixierenden Teils (531) und eine Position eines hinteren Endes (532b) des zweiten dazwischenliegenden fixierenden Teils (532) in der Vorn-Hinten-Richtung verschieden sind.

3. Absorbierender Artikel (1) nach Anspruch 1 oder 2, wobei
eine Länge in der Vorn-Hinten-Richtung (L531) des ersten dazwischenliegenden fixierenden Teils (531) und eine Länge in der Vorn-Hinten-Richtung (L532) des zweiten dazwischenliegenden fixierenden Teils (532) verschieden voneinander sind.

4. Absorbierender Artikel (1) nach einem der Ansprüche 1 bis 3, wobei,
in Bezug auf einen Abstand (d53f) zwischen dem vorderen Ende (531f) des ersten dazwischenliegenden fixierenden Teils (531) und dem vorderen Ende (532f) des zweiten dazwischenliegenden fixierenden Teils (532) in der Vorn-Hinten-Richtung,
der Abstand (d53f) kürzer ist als eine Länge in der Vorn-Hinten-Richtung (L531) des ersten dazwischenliegenden fixierenden Teils (531) und
der Abstand (d53f) kürzer ist als eine Länge in der Vorn-Hinten-Richtung (L532) des zweiten dazwischenliegenden fixierenden Teils (532).

5. Absorbierender Artikel (1) nach einem der Ansprüche 1 bis 4, wobei
in Bezug auf einen Abstand (d53b) zwischen einem hinteren Ende (531b) des ersten dazwischenliegenden fixierenden Teils (531) und einem hinteren Ende (532b) des zweiten dazwischenliegenden fixierenden Teils (532) in der Vorn-Hinten-Richtung,
der Abstand (d53b) kürzer ist als eine Länge in der Vorn-Hinten-Richtung (L531) des ersten dazwischenliegenden fixierenden Teils (531) und
der Abstand (d53b) kürzer ist als eine Länge in der Vorn-Hinten-Richtung (L532) des zweiten dazwischenliegenden fixierenden Teils (532).

6. Absorbierender Artikel (1) nach einem der Ansprüche 1 bis 5, wobei
der Saugkern (11) einen schmalen Teil (11C) in einem mittleren Teil in der Vorn-Hinten-Richtung aufweist,
wobei der schmale Teil (11C) eine laterale Breite aufweist, die kleiner ist als Endteile des Saugkerns (11), und
das vordere Ende (531f) des ersten dazwischenliegenden fixierenden Teils (531) und das vordere Ende (532f) des zweiten dazwischenliegenden fixierenden Teils (532) den schmalen Teil (11C) in der Vorn-Hinten-Richtung überlappen.

7. Absorbierender Artikel (1) nach Anspruch 6, wobei
ein hinteres Ende (531b) des ersten dazwischenliegenden fixierenden Teils (531) und ein hinteres Ende (532b) des zweiten dazwischenliegenden fixierenden Teils (532) den schmalen Teil (11C) in der Vorn-Hinten-Richtung überlappen.

8. Absorbierender Artikel (1) nach Anspruch 6 oder 7, wobei
der Saugkern (11) einen schmalsten Teil (11Cn) in dem schmalen Teil (11C) aufweist,
wobei der schmalste Teil (11Cn) eine laterale Breite aufweist, die in dem schmalen Teil (11C) am kleinsten ist, und
der erste dazwischenliegende fixierende Teil (531) und der zweite dazwischenliegende fixierende Teil (532) den schmalsten Teil (11Cn) in der Vorn-Hinten-Richtung überlappen.

9. Absorbierender Artikel (1) nach einem der Ansprüche 1 bis 8, wobei
der dazwischenliegende fixierende Teil (53) zumindest einen Teil des sich dehnenden/zusammenziehenden Elements (41), das in dem auslaufsicheren Wandteil (40) eingeschlossen ist, in einer Dickenrichtung überlappt.

10. Absorbierender Artikel (1) nach einem der Ansprüche 1 bis 9, wobei
sich der dazwischenliegende fixierende Teil (53) auf einer vorderen Seite in der Vorn-Hinten-Richtung in Bezug auf eine Mitte des Saugkörpers (10) befindet.

11. Absorbierender Artikel (1) nach einem der Ansprüche 1 bis 10, wobei
ein vorbestimmter Bereich (g53o) zwischen einem äußeren Ende des dazwischenliegenden fixierenden Teils (53) und einem äußeren Ende des auslaufsicheren Wandteils (40) in der lateralen Richtung bereitgestellt ist.

12. Absorbierender Artikel (1) nach einem der Ansprüche 1 bis 11, wobei
ein vorbestimmter Bereich (g53i) zwischen einem inneren Ende des dazwischenliegenden fixierenden Teils (53) und einem inneren Ende des auslaufsicheren Wandteils (40) in der lateralen Richtung bereitgestellt ist.

## Revendications

1. Article absorbant (1) comportant :
un corps absorbant (10) comprenant une partie centrale absorbante (11) et une feuille côté orienté vers la peau (12) disposée sur un côté orienté vers la peau par rapport à la partie centrale absorbante (11) ; et
une paire de parties formant paroi antifuite (40) comprenant un élément d'extension/de contraction (41) qui s'étend et qui se contracte,
dans un état dans lequel l'article absorbant (1) est déplié, le corps absorbant (10) ayant une direction allant d'avant en arrière et une direction latérale qui se croisent l'une par rapport à l'autre,
les parties de la paire de parties formant paroi antifuite (40) étant mises en oeuvre sur deux côtés latéraux du corps absorbant (10),
une partie de fixation avant (51) étant mise en oeuvre dans une partie d'extrémité avant du corps absorbant (10) et servant à fixer les parties formant paroi antifuite (40) de telle sorte que les parties formant paroi antifuite (40) sont incapables de s'élever,
une partie de fixation arrière (52) étant mise en oeuvre dans une partie d'extrémité arrière du corps absorbant (10) et servant à fixer les parties formant paroi antifuite (40) de telle sorte que les parties formant paroi antifuite (40) sont incapables de s'élever,
une partie de fixation intermédiaire (53) étant mise en oeuvre entre la partie de fixation avant (51) et la partie de fixation arrière (52) dans la direction allant d'avant en arrière,
la partie de fixation intermédiaire (53) servant à fixer les parties formant paroi antifuite (40) sur la feuille côté orienté vers la peau (12),
la partie de fixation intermédiaire (53) étant mise en oeuvre au niveau d'une position séparée de la partie de fixation avant (51) et de la partie de fixation arrière (52) dans la direction allant d'avant en arrière,
la partie de fixation intermédiaire (53) ayant une première partie de fixation intermédiaire (531) et une deuxième partie de fixation intermédiaire (532),
la première partie de fixation intermédiaire (531) étant disposée sur un côté latéral,
la deuxième partie de fixation intermédiaire (532) étant disposée sur un autre côté latéral,
une position d'une extrémité avant (531f) de la première partie de fixation intermédiaire (531) et une position d'une extrémité avant (532f) de la deuxième partie de fixation intermédiaire (532) étant différentes dans la direction allant d'avant en arrière.

2. Article absorbant (1) selon la revendication 1, dans lequel
une position d'une extrémité arrière (531b) de la première partie de fixation intermédiaire (531) et une position d'une extrémité arrière (532b) de la deuxième partie de fixation intermédiaire (532) sont différentes dans la direction allant d'avant en arrière.

3. Article absorbant (1) selon la revendication 1 ou la revendication 2, dans lequel
une longueur (L531) dans la direction allant d'avant en arrière de la première partie de fixation intermédiaire (531) et une longueur (L532) dans la direction allant d'avant en arrière de la deuxième partie de fixation intermédiaire (532) sont différentes l'une par rapport à l'autre.

4. Article absorbant (1) selon l'une quelconque des revendications 1 à 3, dans lequel
en ce qui concerne une distance (d53f) entre l'extrémité avant (531f) de la première partie de fixation intermédiaire (531) et l'extrémité avant (532f) de la deuxième partie de fixation intermédiaire (532) dans la direction allant d'avant en arrière,
la distance (d53f) est plus courte qu'une longueur (L531) dans la direction allant d'avant en arrière de la première partie de fixation intermédiaire (531), et
la distance (d53f) est plus courte qu'une longueur (L532) dans la direction allant d'avant en arrière de la deuxième partie de fixation intermédiaire (532).

5. Article absorbant (1) selon l'une quelconque des revendications 1 à 4, dans lequel
en ce qui concerne une distance (d53b) entre une extrémité arrière (531b) de la première partie de fixation intermédiaire (531) et une extrémité arrière (532b) de la deuxième partie de fixation intermédiaire (532) dans la direction allant d'avant en arrière,
la distance (d53b) est plus courte qu'une longueur (L531) dans la direction allant d'avant en arrière de la première partie de fixation intermédiaire (531), et
la distance (d53b) est plus courte qu'une longueur (L532) dans la direction allant d'avant en arrière de la deuxième partie de fixation intermédiaire (532).

6. Article absorbant (1) selon l'une quelconque des revendications 1 à 5, dans lequel
la partie centrale absorbante (11) a une partie étroite (11C) dans une partie centrale dans la direction allant d'avant en arrière,
la partie étroite (11C) ayant une largeur latérale inférieure à des parties d'extrémité de la partie centrale absorbante (11), et
l'extrémité avant (531f) de la première partie de fixation intermédiaire (531) et l'extrémité avant (532f) de la deuxième partie de fixation intermédiaire (532) chevauchent la partie étroite (11C) dans la direction allant d'avant en arrière.

7. Article absorbant (1) selon la revendication 6, dans lequel
une extrémité arrière (531b) de la première partie de fixation intermédiaire (531) et une extrémité arrière (532b) de la deuxième partie de fixation intermédiaire (532) chevauchent la partie étroite (11C) dans la direction allant d'avant en arrière.

8. Article absorbant (1) selon la revendication 6 ou la revendication 7, dans lequel
la partie centrale absorbante (11) a une partie la plus étroite (11Cn) dans la partie étroite (11C),
la partie la plus étroite (11Cn) ayant une largeur latérale qui est la plus petite dans la partie étroite (11C), et
la première partie de fixation intermédiaire (531) et la deuxième partie de fixation intermédiaire (532) chevauchent la partie la plus étroite (11Cn) dans la direction allant d'avant en arrière.

9. Article absorbant (1) selon l'une quelconque des revendications 1 à 8, dans lequel
la partie de fixation intermédiaire (53) chevauche au moins une partie de l'élément d'extension/de contraction (41) comprise dans la partie formant paroi antifuite (40), dans une direction allant dans le sens de l'épaisseur.

10. Article absorbant (1) selon l'une quelconque des revendications 1 à 9, dans lequel
la partie de fixation intermédiaire (53) est située sur un côté avant dans la direction allant d'avant en arrière par rapport à un centre du corps absorbant (10).

11. Article absorbant (1) selon l'une quelconque des revendications 1 à 10, dans lequel
un espace prédéterminé (g53o) est mis en oeuvre entre une extrémité extérieure de la partie de fixation intermédiaire (53) et une extrémité extérieure de la partie formant paroi antifuite (40) dans la direction latérale.

12. Article absorbant (1) selon l'une quelconque des revendications 1 à 11, dans lequel
un espace prédéterminé (g53i) est mis en oeuvre entre une extrémité intérieure de la partie de fixation intermédiaire (53) et une extrémité intérieure de la partie formant paroi antifuite (40) dans la direction latérale.
